(19) Europäisches Patentamt
European Patent Office
Office européen des brevets

(11) Numéro de publication : **0 559 571 A1**

(12) # DEMANDE DE BREVET EUROPEEN

(21) Numéro de dépôt : **93400568.7**

(22) Date de dépôt : **05.03.93**

(51) Int. Cl.$^5$ : **C07D 417/06**, C07D 417/14, A61K 31/425, A61K 31/47

(30) Priorité : **06.03.92 FR 9202671**

(43) Date de publication de la demande : **08.09.93 Bulletin 93/36**

(84) Etats contractants désignés : **AT BE CH DE DK ES FR GB GR IE IT LI MC NL PT SE**

(71) Demandeur : **ADIR ET COMPAGNIE**
**1 rue Carle Hébert**
**F-92415 Courbevoie Cédex (FR)**

(72) Inventeur : **de Nanteuil, Guillaume**
**12 rue du Chemin Vert**
**F-92150 Suresnes (FR)**
Inventeur : **Duhault, Jacques**
**14 bis rue Paul Demange**
**F-78290 Croissy Sur Seine (FR)**
Inventeur : **Espinal, Joseph**
**11 rue Chateaubriand**
**F-75008 Paris (FR)**
Inventeur : **Ravel, Denis**
**32 rue de l'Yvette**
**F-91430 Igny (FR)**

(54) **Nouveaux dérivés de thiazolidine-2,4-dione, leur procédé de préparation et les compositions pharmaceutiques qui les contiennent.**

(57) Composés de formule (I) :

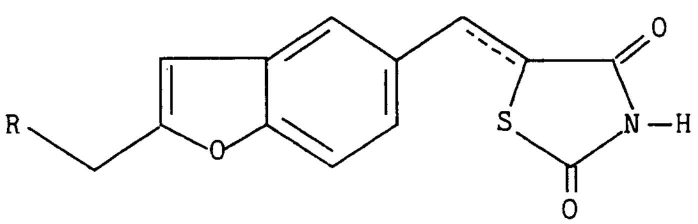

(I)

dans laquelle :
- le trait en pointillés signifie la présence ou non d'une double liaison,
- R représente l'un des groupements A ou B suivants :

A :

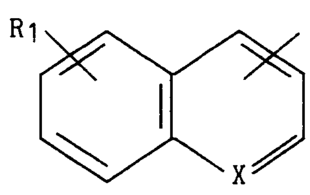

B :

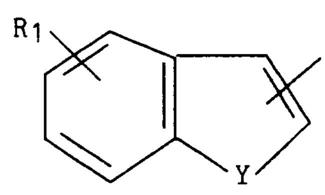

dans lesquels :
- $R_1$ représente un atome d'hydrogène, d'halogène, un groupement alkyle ($C_1$-$C_6$) linéaire ou ramifié, un groupement alkoxy ($C_1$-$C_6$) linéaire ou ramifié,
- X représente un radical CH ou un atome d'azote,
- Y représente un radical NH, un atome d'oxygène ou de soufre, leurs isomères, énantiomères et épimères ainsi que leurs sels d'addition à un acide pharmaceutiquement acceptable.
Médicaments.

EP 0 559 571 A1

La présente invention concerne de nouveaux dérivés de la thiazolidine-2,4-dione, leur procédé de préparation et les compositions pharmaceutiques qui les contiennent.

De nombreux dérivés de la thiazolidine-2,4-dione sont déjà décrits dans l'état de la technique comme agents anti-diabétiques, par exemple dans les brevets EP-A-356214 ou WO-A-86/07056.

Le brevet EP-A-299620 mentionne des dérivés de la benzofuranylméthylthiazolidine-2,4-dione substituée par des groupements phényle ou phényles substitués, pyridyle ou pyridyles substitués et oxazolyle ou oxazolyles substitués.

Dans le brevet WO-A-91/12003 sont décrits des dérivés de la thiazolidinedione connus comme agents antidiabétiques et utilisés pour leur propriété anti-hypertensive chez des sujets insulino-résistants.

Les composés de la présente invention, outre le fait qu'ils soient nouveaux, se différentient des autres dérivés de la thiazolidine-2,4-dione par l'intensité de leurs propriétés pharmacologiques.

En effet, l'insulinorésistance et le défaut de sécrétion de l'insuline sont les responsables de l'intolérance au glucose observée chez les patients ayant un diabète non insulino-dépendant.

Les thérapeutiques actuellement disponibles permettent de corriger essentiellement le défaut de sécrétion d'insuline sans nécessairement améliorer la sensibilité à l'insuline des tissus périphériques (muscles, tissu adipeux).

Les composés appartenant à la structure thiazolidine-2,4-dione sont capables de provoquer une baisse de la glycémie et d'améliorer la tolérance au glucose dans des modèles de diabète non insulino-dépendant sans provoquer une augmentation de sécrétion d'insuline. Nos composés présentent l'avantage d'être particulièrement puissants plus spécialement par rapport à la ciglitazone, composé de référence appartenant à cette structure chimique et dont l'efficacité reste faible.

Ainsi les composés de l'invention sont utilisables dans le traitement des états diabétiques non insulinopéniques, permettant d'obtenir un meilleur contrôle de la glycémie alors que le taux d'insuline circulante diminue. La prévention de cette hyperinsulinémie relative, associée à une diminution des triglycérides circulant sous l'effet de ces produits, peut concourir à une réduction des risques de macroangiopathie.

Ces mêmes composés trouvent de plus une utilisation dans le traitement de l'hypertension chez des sujets présentant une insulino-résistance associée ou non à d'autres anomalies métaboliques.

Plus spécifiquement, la présente invention concerne les dérivés de formule (I) :

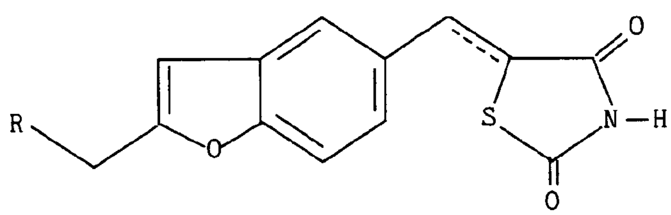

(I)

dans laquelle :

- le trait en pointillés signifie la présence ou non d'une double liaison,
- R représente l'un des groupements A ou B suivants :

A : 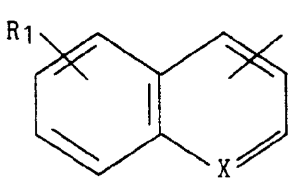

B : 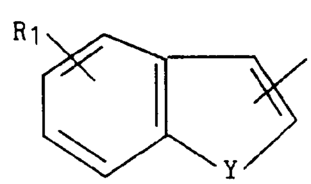

dans lesquels :

- $R_1$ représente un atome d'hydrogène, d'halogène, un groupement alkyle ($C_1$-$C_6$) linéaire ou ramifié, un groupement alkoxy ($C_1$-$C_6$) linéaire ou ramifié,
- X représente un radical CH ou un atome d'azote,
- Y représente un radical NH, un atome d'oxygène ou de soufre,

leurs isomères, énantiomères et épimères ainsi que leurs sels d'addition à un acide pharmaceutiquement acceptable.

L'invention englobe également le procédé de préparation des composés de formule (I), caractérisé en ce que l'on fait réagir un composé de formule (II) :

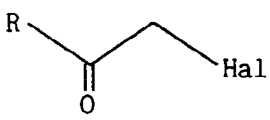

(II)

dans laquelle :

- R est tel que défini dans la formule (I),
- Hal représente un atome de chlore ou de brome,

avec le 5-bromosalicylaldéhyde en présence d'un sel alcalin, par exemple le carbonate de potassium, pour donner (III) :

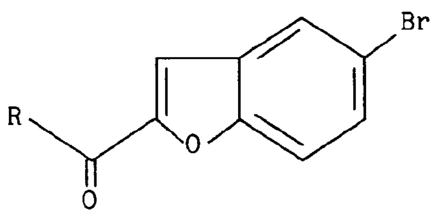

(III)

où R est tel que décrit dans (II) qui, soumis à l'action du triéthyl silane, est réduit en (IV) :

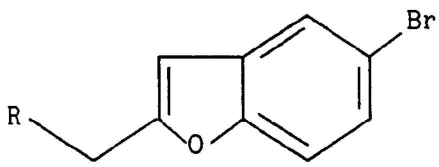

(IV)

formule dans laquelle R est tel que décrit précédemment,
lequel est transformé en composé de formule (V) par le cyanure cuivreux :

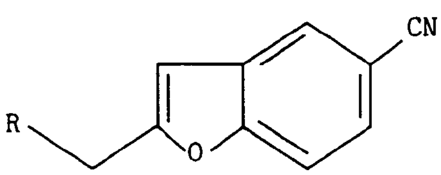

(V)

où R est tel que décrit précédemment, pour donner par réaction avec un amalgame aluminium/nickel en présence d'acide, comme par exemple l'acide trifluoroacétique, le composé de formule (VI), ces deux dernières étapes étant décrites par R.L. Dow et coll. (J. Med. Chem., 34, (1988), 1538-1544) :

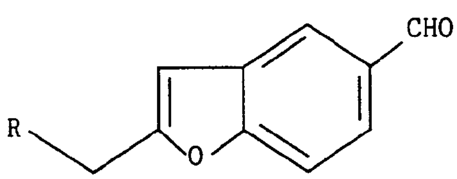

(VI)

dans laquelle R a la même signification que précédemment, lequel composé (VI) est condensé sur la thiazolidine-2,4-dione en présence d'une base, comme la pipéridine, afin d'obtenir le composé (Ia), cas particulier des composés de formule (I) :

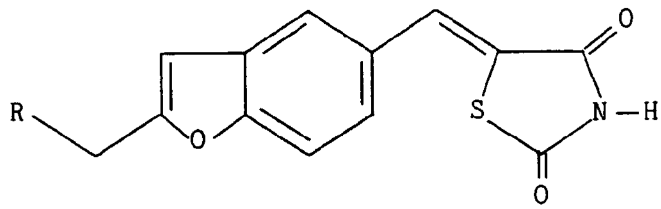

(Ia)

où R est tel que décrit précédemment, qui, par hydrogénation catalytique, conduit au produit (Ib), cas particulier des composés de formule (I) :

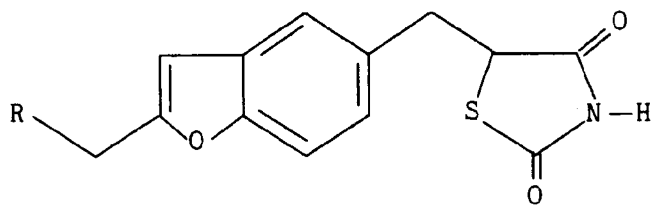

(Ib)

où R est tel que décrit précédemment,
composés de formules (Ia) et (Ib) que l'on purifie le cas échéant selon une technique classique de purification et dont on sépare, si on le souhaite, les isomères par une technique classique de séparation et que l'on transforme, si nécessaire, en leurs sels d'addition à un acide pharmaceutiquement acceptable.

Les composés de formule (I) possèdent des propriétés pharmacologiques très intéressantes. En effet, les dérivés de la thiazolidinedione sont généralement inactifs in vitro et chez les animaux normoglycémiques ne présentant pas d'anomalie de la tolérance au glucose. L'étude pharmacologique des composés de l'invention est donc réalisé :

- dans un modèle de diabète non insulino-dépendant (NIDDM) : la souris ob/ob et
- dans un modèle de tolérance au glucose diminuée, associée à une hyperinsulinémie et une hyperlipémie : le rat Zucker FaFa.

Les résultats obtenus lors de ces tests montrent que les composés de l'invention permettent un contrôle de la glycémie alors que les taux d'insuline circulante et de triglycérides diminuent.
En plus de ces propriétés pharmacologiques relatives à l'hyperinsulinémie, les composés de formule (I), bien que dépourvus d'activité hypotensive intrinsèque, diminuent la pression artérielle des sujets insulino-résistants et de ce fait peuvent être utilisés en thérapeutique dans le traitement de l'hypertension associée aux états d'insulino-résistance et d'autres maladies métaboliques telles que par exemple obésité, dyslipémie, hyperinsulinémie, etc... qui constituent des facteurs de risques cardiovasculaires importants (coronaropathies, macroangiopathie, etc...).

La présente invention a également pour objet les compositions pharmaceutiques contenant comme principe actif au moins un dérivé de formule (I) ou un de ses sels physiologiquement tolérables, seul ou en combinaison avec un ou plusieurs excipients pharmaceutiquement appropriés.

Les compositions pharmaceutiques de l'invention ainsi obtenues sont présentées avantageusement sous formes convenant pour l'administration orale, parentérale ou nasale comme par exemple les comprimés (simples ou dragéifiés), les comprimés sublinguaux, les gélules, les tablettes, ainsi que sous formes de suppositoires, de crèmes, pommades, gels dermiques, etc...

La posologie utile varie selon l'âge et le poids du patient, la nature et la sévérité de l'affection, la voie d'administration et selon la nature des traitements associés. De manière générale, la posologie s'échelonne de 50 à 1000 mg de principe actif de l'invention, en 1 à 3 prises par 24 heures.

Les exemples suivants illustrent la présente invention sans toutefois la limiter en aucune façon. Les produits de départ sont connus ou sont préparés selon des modes opératoires classiques, à partir de matières premières connues.

**EXEMPLE 1 : 5-[2-(2-Naphtylméthyl)-5-benzofuranylméthylène] thiazolidine-2,4-dione**

Première étape : 2-(2-Naphtoyl)-5-bromobenzofurane

186 g (0,74 moles) de 2-bromo-2'-acétonaphtone, 150 g (0,74 moles) de 5-bromosalicylaldéhyde et 103 g (0,74 moles) de carbonate de potassium sont mélangés dans 3,5 litres de cyanure de méthyle ; le tout est ensuite porté à reflux pendant toute une nuit. Le mélange est refroidi à 0°C puis filtré pour donner

220 g d'un solide marron qui est lavé et séché.

Point de fusion : 158-159°C

Deuxième étape : 2-(2-Naphtylméthyl)-5-bromobenzofurane

220 g (0,63 moles) de produit précédemment obtenu sont ajoutés à 1,5 litre d'acide trifluoroacétique et le mélange refroidi à 0°C. 220 ml (1,38 moles) de triéthylsilane sont ajoutés goutte à goutte puis la température maintenue 2 heures à 0°C.

Après évaporation de l'acide trifluoroacétique, le résidu est repris par l'acétate d'éthyle, lavé à l'eau puis avec une solution saturée de chlorure de sodium, avant d'être séché sur sulfate de magnésium. Après évaporation du solvant et purification sur colonne de silice (éluant : cyclohexane/chlorure de méthylène : 95/5), 75 g de produit attendu sont obtenus.

Point de fusion : 98-100°C

Troisième étape : 2-(2-Naphtylméthyl)-5-cyanobenzofurane

75 g (0,22 moles) de produit obtenu dans la deuxième étape sont ajoutés à température ambiante à 40 g (0,44 moles) de cyanure cuivreux dans 620 ml de diméthylformamide. Le mélange est porté à 150°C pendant 48 heures ; 20 g (0,22 moles) de cyanure cuivreux sont alors ajoutés et le mélange est à nouveau porté à 150°C pendant 60 heures.

Le milieu réactionnel, revenu à température ambiante, est versé sur 300 ml d'ammoniaque concentré provoquant une coloration bleue. Après extraction à l'acétate d'éthyle, séchage sur sulfate de magnésium et évaporation des solvants, 60 g d'une huile noire sont obtenus. Une purification sur colonne de silice donne le produit attendu sous forme de solide blanc.

Point de fusion : 202-203°C

Quatrième étape : 2-(2-Naphtylméthyl)-5-formylbenzofurane

21 g (74,2 mmoles) du produit de la troisième étape sont chargés à température ambiante, dans un ballon contenant 22,3 g d'un amalgame aluminium/nickel dans 220 ml d'acide formique à 70 %. L'ensemble est porté au reflux pendant 2 heures 30. Après retour à température ambiante, le milieu réactionnel est filtré et les résidus solides lavés avec de l'acide formique à 70 %.

Le filtrat est extrait à l'acétate d'éthyle et l'amalgame Ni/Al repris plusieurs fois par l'acétate d'éthyle bouillant. Les phases organiques sont lavées avec une solution d'hydroxyde de sodium 4N, jusqu'à une valeur de pH constante et égale à 12. Après lavage à l'eau, ces phases organiques sont séchées et évaporées pour obtenir 19,7 g du produit attendu.

Point de fusion : 97-101°C

Cinquième étape : 5-[2-(2-Naphtylméthyl) 5-benzofuranylméthylène]thiazolidine-2,4-dione

19,7 g (68,8 mmoles) de l'aldéhyde obtenu dans la quatrième étape est mis en présence, à température ambiante de 8,1 g (69,2 mmoles) de thiazolidine-2,4-dione et de 5 ml de pipéridine, dans 650 ml d'éthanol absolu. Le mélange est porté toute une nuit à reflux puis mis à refroidir à température ambiante. Le solvant est évaporé et une huile épaisse est obtenue. Celle-ci est reprise à l'isopropanol dans lequel l'huile précipite.

Le solide obtenu (7,3 g) est filtré puis séché.

Point de fusion : 210°C

**EXEMPLE 2 : 5-[2-(2-Naphtylméthyl)-5-benzofuranylméthyl] thiazolidine-2,4-dione**

5,5 g (14,3 mmoles) de composé obtenu dans l'exemple 1 sont chargés en autoclave à température ambiante avec 5 g de palladium (dispersé à 10 % dans le charbon) dans 80 ml de tétrahydrofurane. L'autoclave est mis en pression à 60 kg et la réaction est effectuée à 60°C pendant 2 jours. Après purification sur colonne de silice et évaporation des solvants, le solide est lavé à l'éther et 530 mg de produit blanc sont obtenus.

Point de fusion : 151-153°C

| Microanalyse élémentaire : | | | | |
|---|---|---|---|---|
| | C % | H % | N % | S % |
| calculé | 71,30 | 4,42 | 3,61 | 8,28 |
| trouvé | 71,12 | 4,43 | 3,95 | 8,05 |

**EXEMPLE 3 : 5-[2-(2-Quinolylméthyl)-5-benzofuranylméthylène] thiazolidine-2,4-dione**

Le produit est préparé selon le même mode opératoire décrit pour l'exemple 1, la première étape étant dans ce cas une réaction entre le 5-bromosalicylaldéhyde et la bromométhyl(2-quinolyl)cétone.

**EXEMPLE 4 : 5-[2-(2-Benzofuranylméthyl)-5-benzofuranylméthylène] thiazolidine-2,4-dione**

Ce composé est préparé selon le mode opératoire de l'exemple 1, en utilisant comme produit de départ la bromométhyl(2-benzofuranyl)cétone.

**EXEMPLE 5 : 5-[2-(2-Indolylméthyl)-5-benzofuranylméthylène] thiazolidine-2,4-dione**

Procédé de préparation identique à celui de l'exemple 1 en utilisant la bromométhyl(2-indolyl)cétone comme produit de départ.

**EXEMPLE 6 : 5-[2-(2-Benzo[b]thiénylméthyl)-5-benzofuranylméthylène] thiazolidine-2,4-dione**

Ce composé est préparé selon le mode opératoire de l'exemple 1 en utilisant comme produit de départ la bromométhyl(2-benzo[b]thiényl)cétone.

**EXEMPLE 7 : 5-[2-(1-Naphtylméthyl)-5-benzofuranylméthylène] thiazolidine-2,4-dione**

Procédé de préparation identique à celui de l'exemple 1, le produit de départ étant la bromométhyl(1-naphtyl)cétone.

Les exemples 8 à 12 suivants présentent les produits hydrogénés des composés des exemples 3 à 7 respectivement. Les hydrogénations sont effectuées selon le mode opératoire décrit dans l'exemple 2.

**EXEMPLE 8 : 5-[2-(2-Quinolylméthyl)-5-benzofuranylméthyl] thiazolidine-2,4-dione**

Obtenu par hydrogénation du composé de l'exemple 3.

**EXEMPLE 9 : 5-[2-(2-Benzofuranylméthyl)-5-benzofuranylméthyl] thiazolidine-2,4-dione**

Obtenu par hydrogénation du composé de l'exemple 4.

**EXEMPLE 10 : 5-[2-(2-Indolylméthyl)-5-benzofuranylméthyl] thiazolidine-2,4-dione**

Obtenu par hydrogénation du composé de l'exemple 5.

**EXEMPLE 11 : 5-[2-(2-Benzo[b]thiénylméthyl)-5-benzofuranylméthyl] thiazolidine-2,4-dione**

Obtenu par hydrogénation du composé de l'exemple 6.

**EXEMPLE 12 : 5-[2-(1-Naphtylméthyl)-5-benzofuranylméthyl] thiazolidine-2,4-dione**

Obtenu par hydrogénation du composé de l'exemple 7.

**EXEMPLES 13 et 14 : Enantiomères de l'exemple 2**

**EXEMPLE 13 : 5-[2-(2-Naphtylméthyl)-5-benzofuranylméthyl]thiazolidine-2,4-dione, isomère $\alpha$**

**EXEMPLE 14 : 5-[2-(2-Naphtylméthyl)-5-benzofuranylméthyl]thiazolidine-2,4-dione, isomère $\beta$**

Les isomères $\alpha$ et $\beta$ du composé de l'exemple 2 ont été séparés par chromatographie chirale dans les conditions suivantes :

Colonne : acier inoxydable, L = 25 cm,
diamètre intérieur : 4 mm

Phase stationnaire : Chiralcel 0J (DIACEL)

Phase mobile : Ethanol/hexane (5/5)
Température : 35°C
Débit : 0,9 ml/min
Détection : UV - 254 nm
Temps de rétention : isomère $\alpha$ : 35 min
isomère $\beta$ : 62 min

## ETUDE PHARMACOLOGIQUE

**EXEMPLE 15 : Etude de l'activité des composés de l'invention sur un modèle de diabète non insulino-dépendant (NIDDM)**

Les animaux (souris ob/ob) sont traités chaque jour, pendant 4 jours, en administrant par voie orale les composés de l'invention en suspension dans une solution à 20 % de gomme du Sénégal.
Avant et après traitement, soit à $J_0$ et à $J_5$, le sang est prélevé par ponction du sinus orbital et la glycémie est déterminée.
Le tableau 1 présente les doses des différents produits à administrer pour obtenir le même effet hypoglycémiant (base 100).

- Tableau 1 : Doses provoquant le même effet hypoglycémiant chez la souris ob/ob -

| | Pioglitazone | Englitazone | CS 045 | Ciglitazone | **Exemple 2** |
|---|---|---|---|---|---|
| Dose (mg/kg/jour) pendant 4 jours | 10 | 10-20 | 150-200 | 50-100 | 5-10 |
| Pouvoir hypoglycémiant | 100 | 100 | 100 | 100 | 100 |

L'étude d'un effet-dose montre que la dose donnant une diminution de la glycémie initiale de 30 % : $ED_{30}$ pour le composé de l'exemple 2 est inférieure à 0,1 mg/kg alors que celle de la pioglitazone est comprise entre 5 et 10 mg/kg. La dose minimum active : DMA montrant une diminution de la glycémie de 10 à 15 % est de 0,1 mg/kg pour le composé de l'exemple 2 alors qu'elle est de 3 mg/kg pour la pioglitazone.

**EXEMPLE 16 : Etude de l'activité des composés de l'invention sur un modèle de tolérance au glucose diminuée associée à une hyperinsulinémie et une hyperlipémie**

Les animaux (rats Zucker Fa/Fa mâles) sont traités, chaque jour, pendant 10 jours en administrant, par voie orale, les composés de l'invention à la dose de 5 mg/kg/jour en suspension dans une solution à 20 % de gomme du Sénégal. Le 11ème jour, les animaux sont sacrifiés et le sang recueilli afin de déterminer la glycémie, les triglycérides plasmatiques et l'insuline immuno-réactive. D'autre part, les animaux sont pesés avant et après traitement.

Dans ces conditions, les composés de l'invention n'ont pas d'influence sur le taux de glucose circulant mais diminuent le taux de triglycérides et d'acides gras libres plasmatiques ainsi que celui de l'insuline immuno-réactive. Cette activité est égale ou supérieure à celle d'autres dérivés de thiazolidinedione de référence.

- Tableau 2 : Etude pharmacologique sur rats mâles Zucker Fa/Fa -

| | Dose (mg/kg/jour) pendant 10 jours | Poids $\Delta$ P % $J_{11}$-$J_1$° | Glycémie (%) | Plasma insuline (%) | TG (%) | AGL (%) |
|---|---|---|---|---|---|---|
| Contrôle | 0 | 100 | 100 | 100 | 100 | 100 |
| Pioglitazone | 5 | 219 | 103 | 61 | 44 | 52 |
| **Exemple 2** | 5 | 250 | 97 | 50 | 35 | 48 |

(TG = triglycérides ; AGL = acides gras libres)

## COMPOSITION PHARMACEUTIQUE

**EXEMPLE 17 : Comprimé : formule de préparation pour 1000 comprimés dosés à 50 mg**

| | |
|---|---|
| 5-[2-(2-Naphtylméthyl)-5-benzofuranylméthyl] thiazolidine-2,4-dione | 50 g |
| Hydroxy propyl cellulose | 2 g |
| Amidon de blé | 10 g |
| Lactose | 100 g |
| Stéarate de magnésium | 3 g |
| Talc | 3 g |

## Revendications

1. Composés de formule (I) :

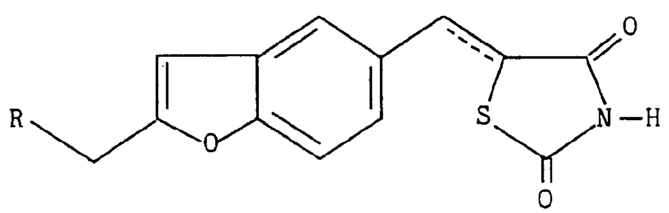

(I)

dans laquelle :
- le trait en pointillés signifie la présence ou non d'une double liaison,
- R représente l'un des groupements A ou B suivants :

A :

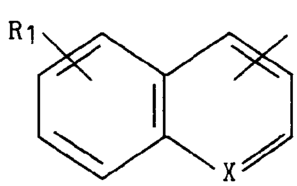

B :

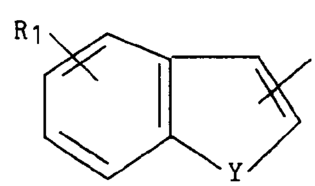

dans lesquels :
- $R_1$ représente un atome d'hydrogène, d'halogène, un groupement alkyle ($C_1$-$C_6$) linéaire ou ramifié, un groupement alkoxy ($C_1$-$C_6$) linéaire ou ramifié,
- X représente un radical CH ou un atome d'azote,
- Y représente un radical NH, un atome d'oxygène ou de soufre,

leurs isomères, énantiomères et épimères ainsi que leurs sels d'addition à un acide pharmaceutiquement acceptable.

2. Composés de formule (I) selon la revendication 1, dans laquelle le groupement R représente le radical naphtyle, leurs isomères, énantiomères et épimères, ainsi que leurs sels d'addition à un acide pharmaceutiquement acceptable.

3. Composé de formule (I) selon la revendication 1 qui est le 5-[2-(2-naphtylméthyl)-5-benzofuranylméthyl]thiazolidine-2,4-dione, ses énantiomères et épimères ainsi que ses sels d'addition à un acide pharmaceutiquement acceptable.

4. Procédé de préparation des composés de formule (I), caractérisé en ce que l'on fait réagir un composé de formule (II) :

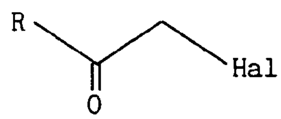

(II)

dans laquelle :
- R est tel que défini dans la formule (I),
- Hal représente un atome de chlore ou de brome,

avec le 5-bromosalicylaldéhyde en présence d'un sel alcalin, par exemple le carbonate de potassium, pour donner (III) :

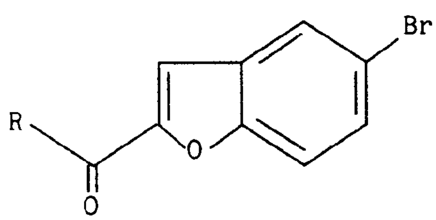

(III)

où R est tel que décrit dans (II) qui, soumis à l'action du triéthyl silane, est réduit en (IV) :

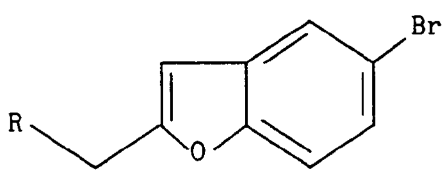

(IV)

formule dans laquelle R est tel que décrit précédemment,
lequel est transformé en composé de formule (V) par le cyanure cuivreux :

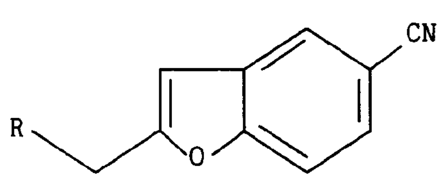

(V)

où R est tel que décrit précédemment, pour donner par réaction avec un amalgame aluminium/nickel en présence d'acide, comme par exemple l'acide trifluoroacétique, le composé de formule (VI) :

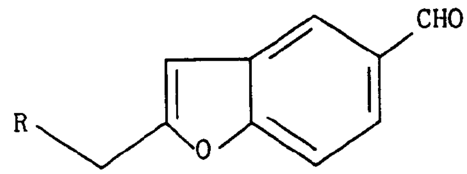

(VI)

dans laquelle R a la même signification que précédemment, lequel composé (VI) est condensé sur la thiazolidine-2,4-dione en présence d'une base, comme la pipéridine, afin d'obtenir le composé (Ia), cas particulier des composés de formule (I) :

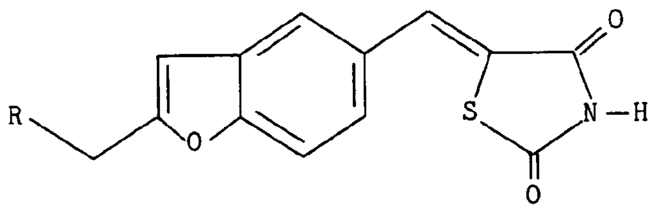

(Ia)

où R est tel que décrit précédemment, qui, par hydrogénation catalytique, conduit au produit (Ib), cas particulier des composés de formule (I) :

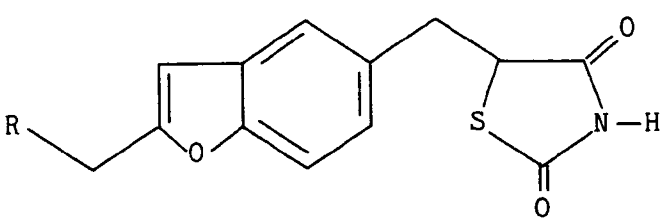

(Ib)

où R est tel que décrit précédemment,
composés de formules (Ia) et (Ib) que l'on purifie le cas échéant selon une technique classique de purification et dont on sépare, si on le souhaite, les isomères par une technique classique de séparation et que l'on transforme, si nécessaire, en leurs sels d'addition à un acide pharmaceutiquement acceptable.

5. Compositions pharmaceutiques contenant comme principe actif au moins un composé selon l'une quelconque des revendications 1 à 3, seul ou en combinaison avec un ou plusieurs véhicules inertes, non toxiques, pharmaceutiquement acceptables.

6. Compositions pharmaceutiques selon la revendication 5 contenant au moins un principe actif selon l'une quelconque des revendications 1 à 3 utiles dans le traitement des diabètes non insulinopéniques associés ou non à une hypertension.

Office européen des brevets

# RAPPORT DE RECHERCHE EUROPEENNE

Numero de la demande

EP 93 40 0568

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,X | EP-A-0 299 620 (BEECHAM GROUP PLC)<br>* revendications *<br>--- | 1,2,4-6 | C07D417/06<br>C07D417/14<br>A61K31/425<br>A61K31/47 |
| X<br><br>D | EP-A-0 207 605 (PFIZER INC.)<br>* revendications *<br>& WO-A-8 607 056<br>----- | 1,2,4-6 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| LA HAYE | 14 AVRIL 1993 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)